# EUROPEAN PATENT SPECIFICATION

(11) **EP 2 261 324 B1**
(45) Date of publication and mention of the grant of the patent: **22.04.2015**
(21) Application number: 10165451.5
(22) Date of filing: 09.06.2010
(51) Int. Cl.: A01N 1/02, C12N 5/00

(54) **Process for cell cultivation and cryopreservation**
Verfahren zur Zellkultivierung und Kryokonservierung
Processus pour la culture cellulaire et cryoconservation

(30) Priority: 09.06.2009 EP 09162323
(43) Date of publication of application: 15.12.2010
(73) Proprietor: Helmholtz-Zentrum für Infektionsforschung GmbH, 38124 Braunschweig (DE)
(72) Inventor: Zghoul, Nadia, Dr., 38124 Braunschweig (DE); Lindenmaier, Werner, Dr., 38124 Braunschweig (DE); Dittmar, Kurt, Dr., 38124 Braunschweig (DE)
(74) Representative: Taruttis, Stefan Georg

(56) References cited:
- EP-A2- 0 364 306
- JP-A- 2008 022 822
- JP-A- 2009 027 944
- DATABASE WPI Week 200816 Thomson Scientific, London, GB; AN 2008-C13002 XP002599552 & JP 2008 022715 A (NISSHO KK) 7 February 2008 (2008-02-07)
- Anonymous: "VueLife FEP Bag, Catalog Number: 32-AC (Adherent Culture)"[Online] 2 October 2009 (2009-10-02), XP002599553 American Fluoroseal Corporation Retrieved from the Internet: URL:http://www.cellgenix.com/ex-vivo-thera peutics/pdf/DataSheets/bags/VueLife_AC_32- ac.pdf> [retrieved on 2010-09-07]
- MACKE LARS ET AL: "Evaluating maturation and genetic modification of human dendritic cells in a new polyolefin cell culture bag system." TRANSFUSION APR 2010 LNKD- PUBMED:20003054, vol. 50, no. 4, April 2010 (2010-04), pages 843-855, XP002599554 ISSN: 1537-2995
- LACHMANN K ET AL: "TAILOR-MADE SURFACE COATINGS FOR CELL CULTIVATION IN A CLOSED PLASTIC BAG SYSTEM" 19TH INTERNATIONAL SYMPOSIUM ON PLASMA CHEMISTRY BOCHUM, GERMANY, JULY 26 - 31, 2009,, [Online] 26 July 2009 (2009-07-26), pages 1-4, XP002540535 Retrieved from the Internet: URL:http://www.ispc-conference.org/ispcpro c/papers/360.pdf>
- CELLUZZI CHRISTINA M ET AL: "Dendritic cell culture: a simple closed culture system using ficoll, monocytes, and a table-top centrifuge." JOURNAL OF HEMATOTHERAPY & STEM CELL RESEARCH OCT 2003 LNKD- PUBMED:14594514, vol. 12, no. 5, October 2003 (2003-10), pages 575-585, XP002599555 ISSN: 1525-8165
- ERDMANN MICHAEL ET AL: "Effective clinical-scale production of dendritic cell vaccines by monocyte elutriation directly in medium, subsequent culture in bags and final antigen loading using peptides or RNA transfection." JOURNAL OF IMMUNOTHERAPY (HAGERSTOWN, MD. : 1997) SEP 2007 LNKD- PUBMED:17667530, vol. 30, no. 6, September 2007 (2007-09), pages 663-674, XP009138304 ISSN: 1524-9557
- VALERI C R ET AL: "Effects of the temperature, the duration of frozen storage, and the freezing container on in vitro measurements in human peripheral blood mononuclear cells", TRANSFUSION (BETHESDA), vol. 36, no. 4, 1996, pages 303-308, ISSN: 0041-1132
- UDOH Y ET AL: "Long term viability of cryopreserved cultured epithelial grafts", BURNS, BUTTERWORTH HEINEMANN, GB, vol. 26, no. 6, 1 September 2000 (2000-09-01), pages 535-542, XP000926096, ISSN: 0305-4179, DOI: 10.1016/S0305-4179(00)00038-3

## Description

The present invention relates to a process for selecting and cultivating cells within a culture vessel, especially for use in the production of implants comprising cells, wherein the culture vessel preferably is an elastic culture vessel like a plastic bag, optionally including the induction of differentiation of cells, and further relates to a process for cryopreserving cells cultivated in a culture vessel.

The processes of the invention allow to cultivate cells, especially adherent cells, e.g. mammalian cells, preferably human cells, in a closed sterile cultivation system vessel and to cryopreserve the cultivated cells which are grown to the state of adherence to the vessel while the cells are adhering to the vessel, optionally to additionally select the cells from a mixture of cell types and to cultivate these selected cell types, and/or to cultivate adherently growing cell types concurrent with inducing differentiation of the cells. Due to the use of a cultivation vessel, which preferably is an elastic cultivation vessel like a plastic, e.g. a polyolefinic bag, provided with inlet and outlet ports for exchange of gas and cultivation media, the processes of the invention are suitable for use for the production of cultivated cells, e.g. forming tissue-like layers, e.g. for use in the production of a pharmaceutical composition containing cultivated cells, e.g. for the manufacture of a transplant.

As a specific advantage, the process for cryopreservation of cultivated adhering cells and/or of cultivated adhering tissue allows the storage of the cultivated cells or cultivated tissue at freezing temperatures, while essentially maintaining the structure of the cultivated cells or cultivated tissue, e.g. maintaining the arrangement of cells, e.g. in a tissue-like structure or in layers.

Further, the cultivated adherently grown cells, also termed tissue-like layer, which are produced by the process of the invention are suitable for use as a medicament, especially as transplant, as the cryopreserved tissue-like layer of cultivated cells can be thawed, optionally further cultivated, and transplanted to a patient, with removal of the adhering culture vessel portion preferably before or after transplantation. Accordingly, the invention also relates to the cryopreserved tissue-like layer, which is produced by the process of the invention, for use as a medicament, e.g. as a transplant or as an implant. Preferably, in the embodiments of the invention, the cells are cells homologous to or immunologically compatible to the transplant recipient.

### State of the art

DE 10 2006 036536 describes a process for modifying the inner surface of a medical cultivation bag by treatment with a plasma in the presence of a reactive compound, e.g. in the presence of a silane, an acid anhydride, or an acrylate, for generating silyl groups, carbon acid groups, or epoxy groups, respectively, on the polymer surface of the culture vessel.

Koller et al. (Biomaterials 1963 - 1972 (1998)) describe that culture devices used for cultivating bone marrow mononuclear cells, the plastic surface can be pretreated to modify its roughness.

Jiao et al. (Biomed. Mater. 2, R24 - R37 (2007)) describe that the modification of polyester surfaces for introducing morphological modifications or surface charges or surface biomacromolecule modification influence biocompatibility of biomaterials for tissue engineering.

JP 2009027944 A according to the English abstract provides a bag-shaped container for cultivating adhesive cells.

JP 20060195426 A according to the English abstract describes the cultivation of a cell-sheet by cultivation of cells in a container made of film, and peeling the cell-sheet from the film.

JP 2008022822 A according to its English abstract describes a cultivation system for cultivation and separation of adult stem cells, the system comprising a means for selectively capturing adult stem cells from a liquid which is pumped into the means, and an outlet for draining liquid out of the means so that cells that have been caputured in the means are then cultivated.

The English abstract of JP 2008 022822 describes a cultivation vessel which has surface-bound ligands that specifically bind adult stem cells from a liquid. The specifically bound stem cells can be cultivated within the vessel.

### Objects of the invention

It is an object of the present invention, especially for use in the production of cultivated cells and/or of cultivated tissue to be used as an implant, to provide a process for selecting and cultivating desired cells from a mixture of cells, e.g. from a biopsy, and to provide a process for cryopreserving cultivated cells and/or cultivated tissue, which cryopreservation process essentially maintains the arrangement of cells generated during the cultivation. Preferably, these processes should be suitable for realisation in plastic cultivation vessels, preferably within a single plastic cultivation vessel.

### General description of the invention

The invention attains the above-mentioned objects by providing the processes as defined in the appended claims, and especially by providing in a first embodiment a process for the production of crypreserved cells comprising the steps of cultivating cells within a plastic cell cultivation vessel, preferably an elastic bag, the vessel having one or more inlet and outlet ports, or one port for inlet and outlet, for introducing cultivation media and medium supplements, and for withdrawal of medium, and the step of adding cryoprotectant and cooling the adherent cells, which can be adherently grown cells, to freezing temperatures, e.g. to at least -20°C, at least -80°C, or in liquid nitrogen. Cooling, it is preferred to cool the cells adherent to at least a section of the cultivation vessel gradually to 0°C or below, e.g. at a rate of 2 to 10°C per minute, or by shock-freezing, e.g. by deposition into liquid nitrogen. Further, the cultivation vessel has inlet and outlet ports for introducing gas required for cultivation, which can be embodied in the form of at least a fraction of the vessel wall being permeable to gas, especially to oxygen and carbon dioxide. In this first embodiment, the cultivation vessel on its inner surface is at least partially provided with or covered by a cell surface binding ligand, which preferably is a proteinaceous binding ligand, e.g. with an antibody and/or a ligand which is specific for a cellular receptor or a cellular receptor specific for a cell surface receptor ligand. According to the invention, the cells are adherently growing cells, and the cells are cultivated under cell culture conditions to a state of adherent growth, e.g. until cells are grown adherently to the cultivation vessel surface, preferably to a state, in which the cultivated cells are confluent or to a state, in which the cells form a tissue-like layer, e.g. to a layer thickness of at least 2 to at least 10 cells.

For the purposes of the present invention, the term "cell" refers to animal cells, especially to mammalian cells, most preferably to human cells, and to respective animal, mammalian and human tissue, preferably selected from the group comprising stem cells, especially bone marrow stem cells, mesenchymal stem cells, including natural progenitor cells and differentiated cells, e.g. endothelial cells, bone marrow cells, osteogenic cells, fat cells, and neuronal progenitor cells. Generally, cells used in the invention are adherently growing cells. Preferably, the cells are ectodermally derived cells or cells of ectodermal origin, e.g. nerve cells, or endodermal cells, e.g. surface cells of the gastrointestinal tract, including liver and pancreas cells, cells of the respiratory tract and cells of an endocrine gland, or the cells are mesodermally derived cells or cells of endodermal origin, e.g. connective tissue cells. Culture vessels for use in the processes of the invention, especially for the cultivation process involving the step of selecting and/or differentiating cells, have a polymeric surface, e.g. a polyolefinic surface, which is provided with hydrophilic and/or positively charged groups, which support adherent growth, and to which groups preferably a cell surface binding ligand is coupled, e.g. by adhesion or chemical linkage. The introduction of hydrophilic and/or positively charged groups to a polymer surface can for example be obtained by contacting the culture vessel surface with a precursor molecule, e.g. tetramethoxy silane, amino propyl trimethoxysilane or diamino cyclohexane or hexamethyl cylotrioxy siloxane in an inert gas atmosphere and inducing a plasma by electrical discharge. Further, the hydrophilic groups can be carboxyl groups, e.g. generated on the vessel's surface by plasma discharge, or by contacting the culture vessel surface with a precursor molecule that contains at least one group reactive with the vessel and one carbon acid, carbon ester or carbon acid anhydride group. Preferably, the hydrohilic and/or positively and/or negatively charged groups are introduced to the vessel's surface by contacting this surface with a percursor molecule during electrical plasma discharge treatment.

When introducing cells into the culture vessel, e.g. suspended cells obtained from a biopsy, the cells used in the invention adhere to the cultivation vessel's surface, proliferate and/or differentiate. For cultivation, the cultivation vessel can be moved for improving mass transfer, and fresh gas, e.g. air with 5% CO₂ can be introduced by an inlet port, and exhaust gas can be withdrawn from the vessel via an outlet port, as generally preferred for the processes of the invention. Inlet and outlet ports for gas exchange can be replaced by gas-permeable walls of at least a section of the culture vessel. When cultivating the cells, e.g. with providing agitation by movement of the culture vessel, adherent cells, especially mesenchymal stem cells, grow to confluence, i.e. the cells form a layer on the vessel's surface.

It has been found that cultivated cells, e.g. grown to at least partial confluence, can be cryopreserved within the cultivation vessel, preferably in combination with adding a cryoprotectant, and with optional removal of the cultivation medium, followed by freezing of the cultivation vessel including the adherent cells. Thawing, preferably followed by the introduction of fresh cultivation medium, preferably including the dilution or removal of cryoprotectant to non-detrimental concentration, results in a viable adherent cultivated cell layer, which essentially has the structural integrity of the cell layer as cultivated. It has been found that cryopreservation of cultivated cells adhering to the culture vessel within the culture vessel results in the maintenance of the structural integrity of the cell layers, and hence in the minimization of damage to cultivated cells by freezing and thawing. Accordingly, the invention provides for a cryopreservation process of cultivated cells or tissue adhering to the surface of a cultivation vessel, which results in the preservation of the original structure of the cellular interconnection, e.g. in the preservation of the cultivated cell layer structure or cultivated tissue structure.

Further, it has been found that the coupling of at least one cell surface binding ligand to the surface of the cultivation vessel results in the selective binding of cells to the cultivation vessel. Accordingly, introduction of an admixture of cell types suspended in a medium, preferably in a cultivation medium, in this second embodiment results in the preferential binding of a cell type to the cultivation vessel surface via the cell surface binding ligand. Removal of unbound cells, e.g. by a simple exchange of medium, essentially results in an enrichment or isolation of cells that specifically bind to the cell surface binding ligand.

When subjecting the cultivation vessel to culture conditions, i.e. when cultivating the cells bound to the cultivation vessel, the enriched or isolated cell type will expand, resulting in an efficient isolation and cultivation process which is performed in a closed system, i.e. in a cultivation vessel which is equipped with inlet ports and outlet ports for sterile culture conditions. Therefore, this process allows the isolation of cells from a mixed suspension of cell types and the subsequent cultivation of the isolated cells within one closed vessel, which preferably is an elastic bag.

The cryopreservation process of the invention can be applied to cells isolated and cultivated within a cultivation vessel, resulting in the production of a cryopreserved cultivated cell layer within one cultivation vessel, i.e. without the need to transfer or otherwise handle the type of cells that are isolated and cultivated from one container to another. Especially for use in the production of cell layers or cell tissue for implantation for medical purposes, the use of closed vessels and the minimisation of handling or transfer steps of the desired type of cells is of advantage, both for ease of performing the process and for maintaining sterile conditions, while also avoiding damage to cells, as e.g. caused by liquid handling steps like pipetting.

When producing a transplant by the cultivation and cryopreservation process, it is generally preferred that the adherently growing cells are immunologically compatible to the transplant reciepient, preferably autologous cells of the transplant recipient. In this embodiment, the process preferably comprises the step of thawing the cultivated tissue layer adhering to at least a section of the cultivation vessel, and removing the section of the cultivation vessel from the tissue layer, e.g. prior to or after use as a transplant.

Preferred cell surface binding ligands are proteinaceous compounds, including e.g. antibodies, especially antibodies having a specificity for a cell types specific cellular surface bound molecule, e.g. anti-CD 32, anti-CD 64, anti-CD20 (specific for B-cells), anti-CD4 in combination with anti-CD8 (specific for T-cells), anti-CD 14 (specific for monocytes and dendritic cells), and/or anti-CD105 in combination with anti-CD73 and anti-CD90 (specific for MSC). Further preferred cell surface binding ligands are natural or synthetic ligands to cell surface receptors, e.g. selected from the group comprising oligomeric glycosyl moieties, e.g. mannose-containing glycosylation moieties, LPS (lipo polysaccharide), members of the TNF-super family (tumor necrosis factor super family), and NGF (nerve growth factor).

For coupling of cell surface binding ligands to the chemically modified polymer surface of the cultivation vessel, preferably to the hydrophilic and/or positively charged substituents of the polymeric vessel surface, unspecific or specific protein adsorption can be employed, e.g. for binding antibodies, cellular receptors, cellular receptor ligands. Preferably, covalent coupling of the cell surface binding ligands to the reactive substituents groups of the polymeric vessel surface can be used for forming a linkage, a covalent linkage of the cell surface binding ligand or a covalent linkage of a portion of a linkage group or compound, to which the cell surface binding ligand is bound.

Alternatively or additionally, an intermediate coupling agent, e.g. polyethylene glycol, biotin, and activated ester like sulfo-NHS-LC-biotin that is reactive to amino groups, can be reacted with the reactive substituents of the vessel surface, and then be contacted with the cell surface binding ligand. Accordingly, the cell surface binding ligand can be modified by introduction of a reactive group or an intermediate coupling agent, e.g. of biotin or another substituent which is co-reactive with a reactive substituent coupled to the vessel surface, and/or co-reactive with the intermediate binding agent attached to the vessel surface.

It could be shown that the introduction of a cell suspension of different cell types suspended in an aqueous medium, e.g. in cell culture medium into a cultivation vessel, which preferably is a polyolefinic bag, e.g. a polyethylene or polypropylene bag equipped with one inlet port for medium, which was also suitable for withdrawing medium, into a cultivation vessel coated with an antibody directed against a cell surface antigen resulted in the enrichment or isolation of the cell-type bearing the antigen by adsorption to the antibody, and, following removal of medium with unbound cells, in the cultivation of the isolated cell-type. Following isolation of cells by adsorption to a cell-surface binding ligand bound to the vessel, with subsequent removal of medium containing unbound cells, optionally followed by cultivation of cells in added medium, adherent cells of the cell-type bearing the antigen to which the cell-surface binding ligand is specific, could be cryopreserved. As an example, a bag was used which on its inner surface was at least partially coated with a bound anti-CD20 antibody. Addition of a mixed cell population (peripheral blood or mononuclear cells isolated from peripheral blood) resulted in the isolation and cultivation of the cell type (B-cells) bearing the respective antigen, i.e. CD20, respectively. Exchange of air/CO₂ and of exhaust gas was found sufficient across the vessel walls of the cell culture bags used (polypropylene bags).

In a further embodiment, the process of the invention includes the step of differentiating cells adhering to, and preferably adherently growing on the culture vessel surface during the cultivation. The differentiation can be caused by adding a differentiation factor to the culture medium or, preferably, a differentiation factor can be bound to the culture vessel surface as described for a surface binding ligand. Preferred differentiation factors are selected from the group comprising growth factors and differentiation factors, e.g. BMPs (bone morphogenic protein), FGFs (fibroblast growth factor), VEGFs (vascular endothelial growth factor), NGFs (neuronal growth factors), TLR-ligands (toll-like receptor-ligands), and TNFR-ligands (tumor-necrosis-factor receptor-ligands).

Withdrawal of medium after an initial contacting time and the subsequent introduction of fresh medium resulted in the isolation of the specifically bound cells. Subsequent cultivation, preferably using a agitation of the cultivation bag by rotary shaking, resulted in the formation of a cell layer, which could be confirmed to essentially consist of the cell type specifically bound to the surface binding ligand. Accordingly, for isolation of a cell-type from a mixture of cells, the process preferably includes the removal of medium containing unbound cells and introducing fresh medium, i.e. cell-free medium.

Induction of the differentiation of the cells could be induced by adding a differentiation factor to the cultivation medium, or by providing the differentiation factor bound to the vessel surface, i.e. the vessel surface being provided with a mixture of the surface binding ligand and of the differentiation factor. The isolation of one cell-type from a mixed cell population and the subsequent cultivation and differentiation of the isolated cell-type was shown on the example of MSC using bone marrow or a trabecular bone biopsy, followed by the cryopreservation of the resultant differentiated cell-type.

The addition of a cryoprotectant to the cultivation vessel, preferably in combination with the removal of at least a fraction of the cultivation medium, and followed by cooling of the cultivation vessel to freezing temperatures, e.g. to -70°C down to liquid nitrogen, resulted in the production of a cryopreserved cultivated cell layer, which upon thawing and addition of medium had preserved the integrity of the cellular interconnection structure. This finding demonstrates the general suitability of the processes of the invention for use in cultivating and cryopreserving cultivated cells, and cultivated tissue, each adhering to the inner surface of the cultivation vessel.

In a specific embodiment, the cell surface binding ligand is a viral particle, which can also be referred to as a viral vector, containing in its nucleic acid sequence a transgene, e.g. an expression cassette. The transgene can be used for genetically manipulating cells, which upon contact with the culture vessel surface also contact the viral particle, ensuring that only cells adhering to the vessel surface are genetically manipulated by the viral particle. Especially in combination with binding a cell type specific binding ligand, e.g. an antibody, to the vessel surface in combination with the binding of a viral particle, the process comprises the step of selecting a cell type from an admixture of cells with the genetic manipulation of the selected cells, and cultivating the cells.

For binding viral particles to the vessel surface, the same methods as described herein with reference to binding ligands can be used, especially the binding of biotinylated viral particles to streptavidin, which is linked to the polymer vessel surface by biotinylated amino groups introduced to the polymer surface.

As an alternative to genetic manipulation of cells isolated in the process of the invention by providing nucleic acid (homologous or heterologous to the cells) in the form of nucleic acids packaged in a viral particle, nucleic acids for genetic manipulation of cells bound to the surface of the cultivation vessel were bound to the surface of the vessel, i.e. without a viral particle shell. In this embodiment, nucleic acid, preferably DNA, encoding the transgene was coupled to the surface of the cultivation vessel, either directly or using coupling agents, e.g. bifunctional compounds, e.g. a dicarbon acid or a carbon acid anhydride, preferably a cleavable bifunctional compound, or coupling agents as mentioned herein for use in coupling a binding ligand to the vessel surface, e.g. the streptavidin-biotin coupling group, preferably with streptavidin bound to the vessel surface and using biotinylation of the DNA. Biotinylation of nucleic acids can generally be obtained by incorparation of biotinylated nucleotides during synthesis of oligonucleotides.

### Detailed description of the invention

The invention is now described by way of examples with reference to the figures, wherein
F igure 1 shows micrographs of cultivated HEK293 cells adhering to the surface of a cell culture bag having a surface modified by A) 3-amino propyl trimethoxy silane (APTMS), B) modified by tetra methoxysilane (TMOS), and C) without surface modification, and micrographs of cultivated A549 cells in a cell culture bag, the surface of which is E) modified with APTMS, F) modified with TMOS, and G) without modification, and D) HEK293 cells and H) A549 cells as cultivated in a standard cell culture dish,
F igure 2 shows micrographs of cultivated SAOS2 cells adhering to the surface of a cell culture bag of one supplier (Cellgenix) with surface modification by A) APTMS, B) by TMOS, and C) without surface modification, and micrographs of cultivated SAOS2 cells in a cell culture bag of another supplier (Miltenyi Biotech), the surface of which is E) modified by APTMS, F) modified by TMOS, and G) without modification, and at D) and H) as cultivated in a standard cell culture dish,
F igure 3 shows micrographs of 293LP and MSC cells, respectively, as cultivated in cell culture bags, the surface of which was modified with APTMS, under A) and D) following cultivation and before cryopreservation, under B) and E) following thawing after freezing at -70 °C and in liquid nitrogen, and in C) and F) following subsequent cultivation,
F igure 4 shows micrographs of primary human mesenchymal stem cells derived from human bone marrow A) on the surface modified with APTMS, B) on a surface modified with DACH, C) on a surface modified with TMOS, and D) on a non-modified polymer surface at day 3 of cultivation,
F igure 5 shows micrographs of human mesenchymal stem cells of trabecular bone grown on the surface of a cell cultivation bag with a surface modification using A), E) APTMS, B), F) DACH, C), G) TMOS, and D), H) without modification at day 3 and at day 10 of the cultivation, respectively,
F igure 6 shows a representation of the cell growth A) of mesenchymal stem cells on the surface of a polymer cultivation bag modified as indicated, at days 3, 7, 10 and 14, and B) the growth of an endothelial progenitor cell line EA.hy 926 of the surface modified cell culture bags as indicated, in comparison to an untreated, i.e. pure olefinic polymer surface, and in comparison to a cell culture plate (culture dish),
F igure 7A shows scanning electron micrographs of human mesenchymal stem cells after cultivation in a cultivating bag, modified with APTMS,
F igure 7B shows an immuno-fluorescence micrograph of neuronal stem cells grown on APTMS-modified polyolefin,
F igure 7C shows an immuno-fluorescence micrograph of neuronal stem cells grown on DACH-modified polyolefin,
F igure 7D shows an immuno-fluorescence micrograph of cardiomyocyte cells grown on DACH-modified polyolefin,
F igure 8 shows micrographs of human primary bone marrow cells, which could be isolated from a suspension of different cells by selective adherence to A) a polymer cell culture bag modified with APTMS, and B) on a standard cell culture flask for comparison,
F igure 9 schematically shows A) and B) the coupling of an antibody to the surface of a cell culture vessel using binding agents, and C) the specificity of binding of the antibody by linkage of the binding agents (left hand column), in comparison to the binding of the antibody on a non-modified polymer culture vessel (right hand columns),
F igure 10 schematically shows A) a model of the selective binding of cells to the cultivating vessel from an admixture of cells, and B) a micrograph of fluorescence-labelled cells in admixture prior to exchange of the medium, and C) a micrograph of the surface bound cells after a change of the medium,
F igure 11 shows a schematic representation of the experimental setup for selective coupling of viral particles using biotinylated viral particle protein,
F igure 12 shows the effect of the coupling of biotinylated viral particles in comparison to non-biotinylated viral particles on the genetic manipulation of cells cultivated according to the invention,
F igure 13 schematically shows a method for binding DNA to the surface of a culture vessel for genetic manipulation of cells bound to the culture vessel,
F igure 14 schematically shows the process for genetic manipulation of cells adhering to the culture vessel by DNA bound to the culture vessel, and
F igure 15 shows micrographs of calcium deposition from mesenchymal stem cell derived cells, in A) after cultivation in osteogenic medium and B) after cultivation in proliferation medium.

### Example 1: Cultivation of cells and cryopreservation of cultivated cell layers

A variety of cells was cultivated within a culture vessel in the form of an elastic plastic bag of synthetic olefinic polymer, e.g. polypropylene (obtainable from Cellgenix or Miltenyi Biotech) following the introduction of amino groups onto the inner vessel surface by treatment with 3-amino propyl trimethoxysilane (APTMS) or tetramethoxysilane (TMOS) for generating silyl groups. For the introduction of the surface modification, the bags were filled with the precursor compound and helium as an inert gas, followed by the generation of a plasma by electrical discharge in the closed bag. For comparison, cells were cultivated in the olefinic culture bags without pretreatment of the surface, and in standard cell cultivation dishes.

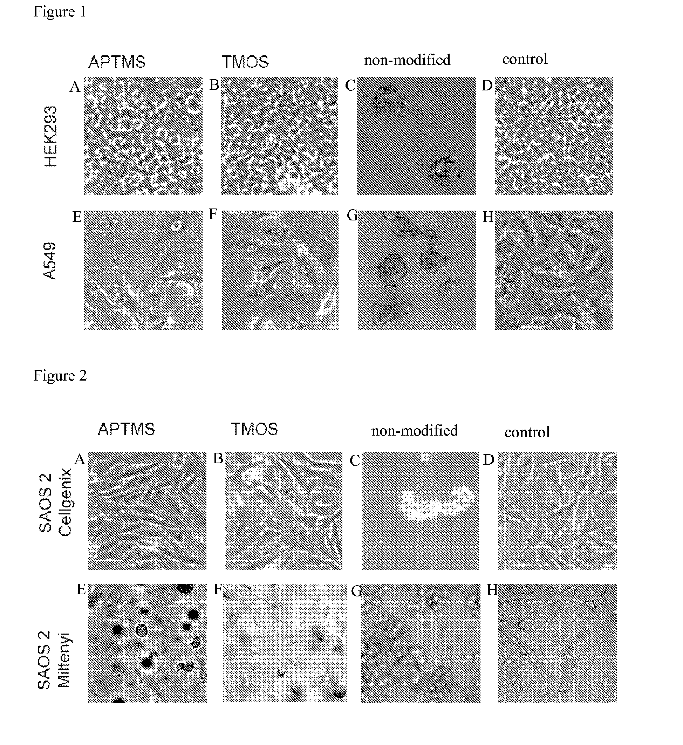

Figure 1 shows that HEK293 cells (human embryonic kidney cell line) during an incubation period of days at 37°C in a 5% CO₂ atmosphere grow to confluence on A) APTMS - treated polymer surfaces, and on B) TMOS - treated polymer bags, whereas essentially no adherent growth can be seen in C) the culture bag with an untreated olefinic surface.
Similarly, A549 cells (a human lung carcinoma cell line) shows adherent growth on E) the APTMS-treated bag surface, and F) on the TMOS- treated bag surface, whereas G) in the bag with non-modified polymer surface, no adherent cell growth is detected.

The control cultures in cultivation dishes of D) HEK293 cells and H) A549 cells show adherent growth of cells comparable to the adherent growth in the culture bags treated with APTMS and TMOS, respectively.

Similarly, growth of the human osteosarcoma cell line SAOS2, which is known to have a limited potential for differentiation, can be grown to confluence on surface treated culture bags to an extent comparable to growth in standard culture dishes, whereas essentially no adherent growth can be seen for non-modified polymer surfaces. In detail, Figure 2 shows the growth of SAOS2 cells after 3 days of cultivation in standard medium (DMEM, 10% FCS) at 37°C in a 5% CO₂ atmosphere at A) in the APTMS- treated culture bag (obtained from Cellgenix), at B) in the TMOS - treated culture bag (Cellgenix) in comparison to the untreated bag at C).

Interestingly, the use of culture bags from a different supplier showed that culture vessels from a different supplier also provided a suitable polymer culture bag, as E) the APTMS - treated culture bag (obtained from Miltenyi) and F) the TMOS - treated culture bag (Miltenyi) yielded adherent growth, but to a reduced extent. Growth shown in C) and G) for the non-modified culture bags_and both control cultures in standard culture dishes, shown at D) and H), showed significantly reduced and normal adherent cell growth, respectively.

These results demonstrate that the process for cultivating cells in a plastic culture bag of olefinic polymer without charged surface groups requires the introduction of modifying groups, preferably of silyl groups or of amino groups to allow for adherent cell growth.

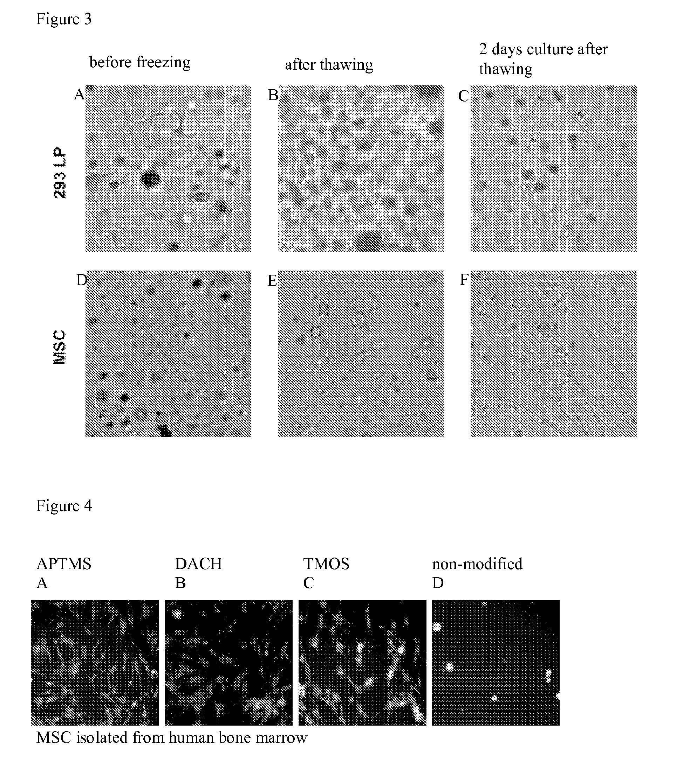

As shown for 293LP cells and for human mesenchymal stem cells (MSC) isolated from bone, cells cultivated in an olefinic polymer culture vessel with surface modification, e.g. in a culture vessel having an olefinic polymer surface containing hydrophilic or positively charged substituent groups, preferably silyl or amino groups, can be cryopreserved within the culture vessel while maintaining the cell structure of a cell layer, and thawing results in a viable cultivated cell layer, which is adherent to the modified olefinic polymer surface. For 293LP cells, the cell layer generated during a cultivation for 3 days up to 70% confluence in MEM with 10% FCS (fetal calf serum) is shown in Figure 3 A). For cryopreservation, the cultivation medium was replaced by a cryoprotectant, containing 90% FCS, 10% DMSO. For cryopreservation, the cultivated cell layers covered in the cryoprotectant and adherent to the cell culture vessel were cooled by incubating at -70 °C overnight, and then transferred to liquid nitrogen. For thawing, the cryopreserved culture was warmed by incubation in a 37°C incubator, and the cryoprotectant was replaced by fresh cultivation medium. Alternatively, cryoprotectant could be diluted to a non- detrimental concentration by adding culture medium.

Figure 3 B) shows a micrograph of the 293LP cells immediately following thawing and after the exchange of the cryoprotectant by fresh culture medium. It can be seen that the cell layer structure is essentially intact, demonstrating that the integrity of the cellular interconnection within the cultivated cell layer is maintained by this cryopreservation process.

Figure 3 C) shows a micrograph of the thawed culture after a further incubation for 2 days at 37°C in a 5% CO₂ atmosphere. This demonstrates that the cryopreserved cells are viable and can be cultivated further in an adherent state.

On the example of bone mesenchymal stem cells (MSC), micrographs of Figure 3 D) for the cultivated cell layer cultivated in DMEM with 15% FCS, in Figure 3 E) after cryopreservation in cryoprotectant (80% FCS, 10% DMEM, 10% DMSO) at -70 °C, and subsequently in liquid nitrogen, followed by thawing by incubation in a 37°C incubator, and replacing the cryoprotectant by the culture medium, and subsequently after an additional 2 days of cultivation in Figure 3 F) at 37°C in a 5% CO₂ atmosphere show that the process for cultivating and cryopreserving cells according to the invention in a culture vessel having an inner surface of an olefinic polymer provided with hydrophilic or positively charged substituent groups is also suitable for stem cells.

### Example 2: Cultivation and cryopreservation of primary human MSC

As described for example 1, cells were cultivated on polyolefinic culture vessel surfaces, which were modified using a plasma for introducing positively charged and hydrophilic substituents. Polymer culture bags (Miltenyi) were surface modified by 3 - amino propyl trimethoxysilane (APTMS), 1, 2 - diamino cyclohexane (DACH), which introduce amino groups, and with tetramethoxy silane (TMOS), which introduces silyl groups to the olefinic surface, as could be shown in analysis.

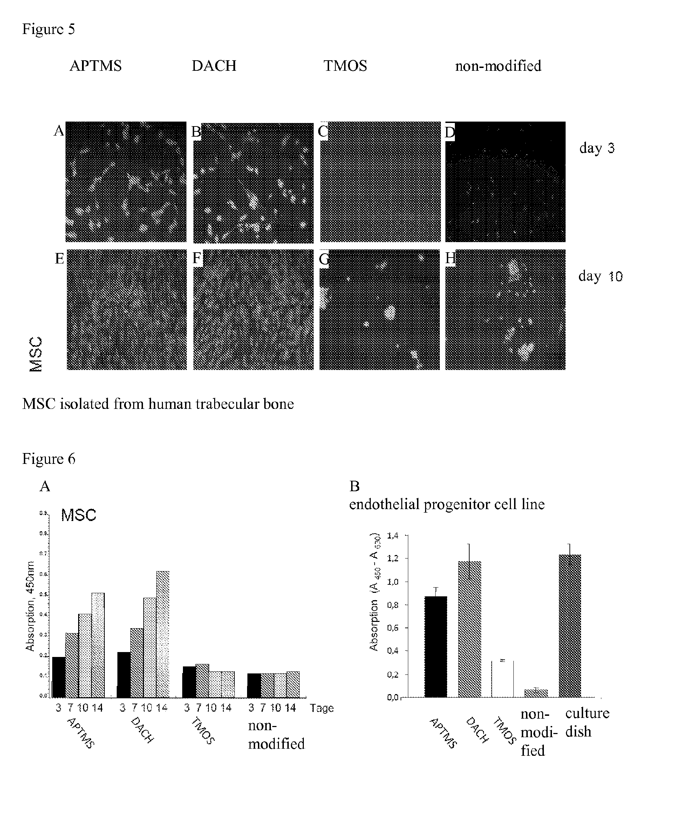

As examples for primary human stem cells, MSC from human bone marrow and MSC from trabecular bone were used. The micrographs of Figures 4 and 5 show fluorescence of cells (bright spots), after staining with the fluorescent dye DIOC6 (available from Invitrogen).

The results of cultivation in standard cell culture medium at 37°C in a 5% CO₂ atmosphere are shown in Figure 4 for the MSC of bone marrow after 3 days of cultivation in A) APTMS-modified culture bags, B) DACH- modified culture bags, and C) TMOS- modified culture bags, and for comparison D) in non-modified culture bags.

Results for MSC from trabecular bone are shown in Figure 5, after cultivation in A) and E) APTMS- modified culture bags, B) and F) in DACH- modified culture bags, and C) and G) in TMOS- modified culture bags, and for comparison D) and H) in non-modified culture bags at day 3 and day 14, respectively. At day 14 of the cultivation, cells grown on D) the APTMS-modified culture bag and in the F) DACH- modified culture bag showed growth, whereas C), G) TMOS - modified culture bags only showed limited adherent growth, and D), H) non-modified culture bags did not show adherent cell growth.

By cryopreserving cultivated cell layers of the MSC of human bone marrow and of human trabecular bone, respectively, as described in Example 1, cryopreservation and thawing yielded viable adherent cultivated cells, which after effective removal of the cryoprotectant could further be cultivated.

The microscopic results demonstrating the effective growth of cells as shown in Figures 4 and 5 were confirmed by analysing the growth of mesenchymal stem cells isolated from human bone marrow and for the human endothelial progenitor cell line EA.hy926. Results of the analysis of cell growth as quantified by the WST-1 metabolic test confirmed that for mesenchymal stem cells, the polyolefinic elastic culture bag having positively charged substituent groups supported adherent growth. In Figure 6, the results are shown, in A) for MSC of bone marrow at days 3, 7, 10 and 14, respectively, for the modifications as indicated, in comparison to the untreated culture bag (non-modified), and in B) the relative growth of the endothelial progenitor cell line in relation to growth on a standard cell culture dish (culture dish).

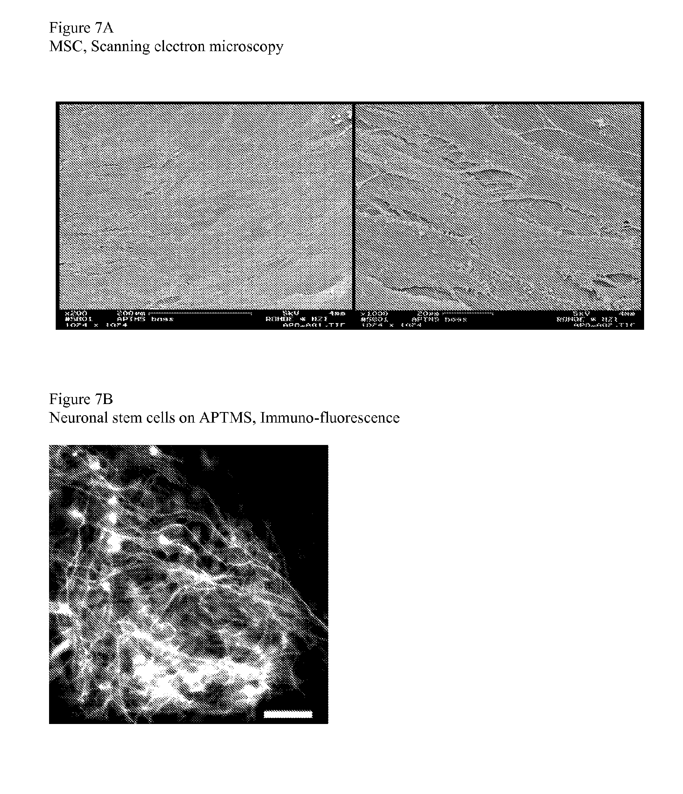

A scanning electron micrograph is shown in Figure 7A for cultivated human mesenchymal stem cells on an olefinic polymer culture bag (Miltenyi), modified to carry positively charged substituent groups (APTMS). The electron micrographs show that the cultivation process of the invention results in a layered cell structure. This cell structure is essentially maintained when cryopreserving the cultivated cell layer as described above, resulting in viable cultivated layered cells, which upon thawing and removal of the cryoprotectant, if detrimental to cell growth, can further be cultivated.

As a further example of cells grown to confluence in a state of adherence, which could be cryopreserved as adherently cultivated cells according to the invention, Figure 7B shows neuronal stem cells derived from the enteric nervous system on an APTMS-modified polyolefinic culture bag. Dyeing was by β-tubulin III.

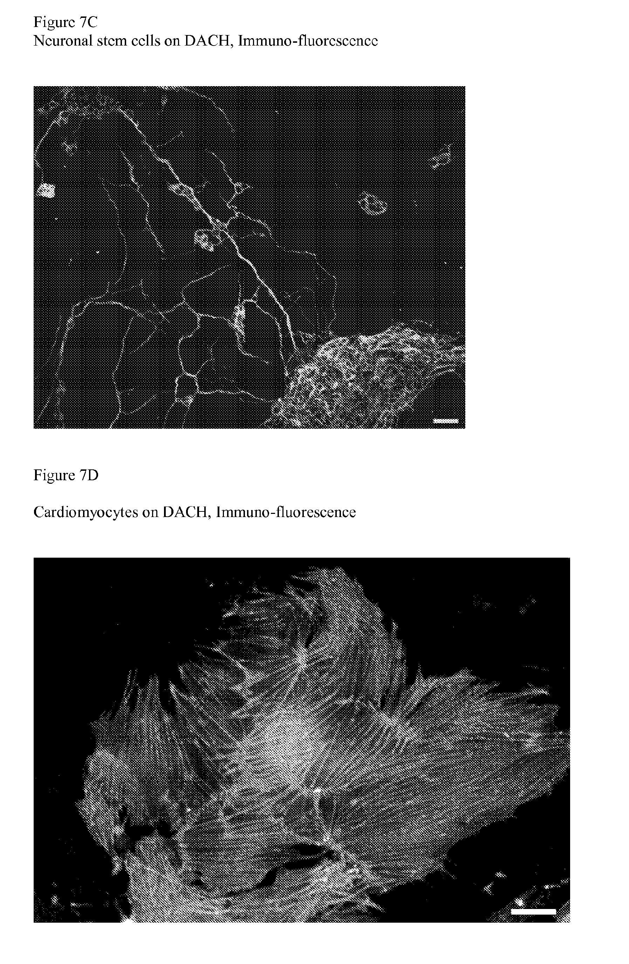

Figure 7C shows neurons from a cell suspension from explanted colon tissue grown on a DACH-modified polyolefinic bag. Dyeing was by β-tubulin III. Length of bar is 40µm.

Figure 7D shows cardiomyocytes on a DACH-modified polyolefinic bag. Dyeing of myofibrils was by anti-α-actinin. Length of bar is 20µm

The adherent cell layers obtained for Figures 7A to 7D could be cryopreserved after adding cryoprotectant while the cell layers were maintained on the hydrophilic polyolefinic surface, and could subsequently be thawed, followed by further cultivation in culture medium, e.g. after removal of cryoprotectant and addition of fresh cultivation medium.

### Example 4: Isolation and cultivation of cells

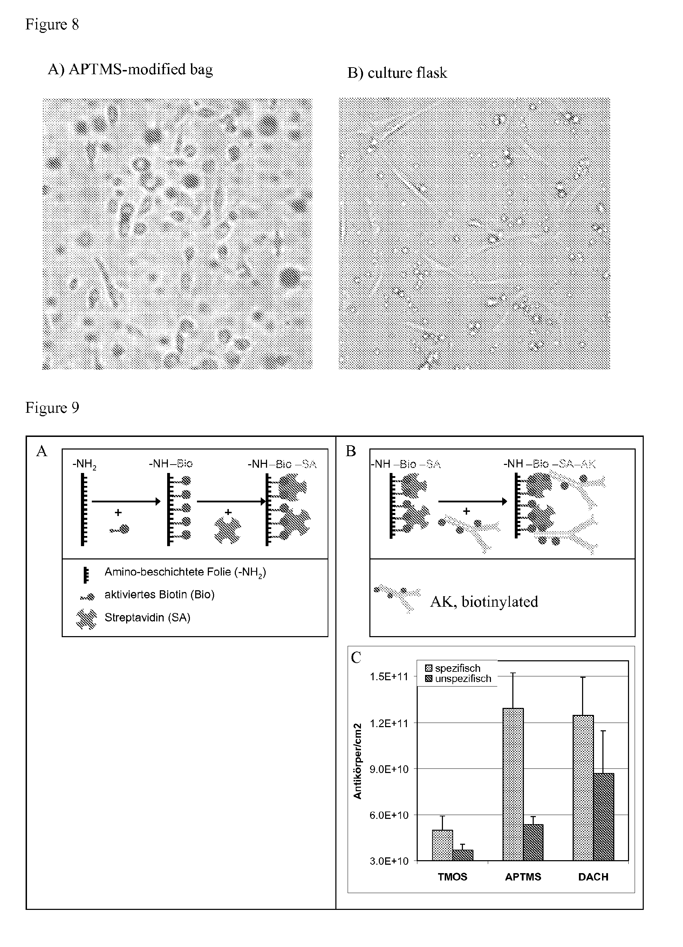

The isolation of one cell type from a mixed cell population, e.g. from a suspension of human bone marrow cells is shown on the example of isolating primary human bone marrow cells using an APTMS - modified polyolefinic culture bag. The mixed cell suspension as prepared in culture medium was introduced into the culture bag and incubated at culture conditions for a period of 12 hours. Subsequently, non - adhering cells were removed by replacing the culture medium by fresh medium, followed by continued incubation under cell culture conditions. As shown in Figure 8A), the microscopic view identifies adherent cells. Under B), a microscopic view obtained in a standard cell culture flask obtained by the standard procedure using the same processing steps is shown.

Generally, this result demonstrates that cells can be isolated from a mixture of cells in suspension by contacting with the cell culture bag having hydrophilic and/or positively charged substituent groups, and incubation, e.g. for 0.5 to 24 h, preferably 2 to 12 h, followed by removal of non-adherent cells, and by cultivation of the adherent cell population.

As described above, the adherent cultivated cells could be cryopreserved within the modified polyolefinic cell culture vessel.

### Example 5: Isolation and cultivation of cells in one culture vessel.

As schematically shown in Figure 9A, a cell surface binding ligand can be bound to the inner surface of a polyolefinic cell culture vessel, e.g. by modifying the polyolefinic surface by introduction of amino groups, e.g. as described above by reacting with APTMS or DACH, and coupling of a biotin containing compound, e.g. activated biotin (Bio) to the amino groups (NH). Using streptavidin (SA), a biotinylated cell surface binding ligand, e.g. an antibody (AK) can be bound to the vessel's surface. Herein, biotin and streptavidin form a linkage group for binding the cell specific binding ligand to the culture vessel at the positively charged or hydrophilic surface groups of the polymer vessel being predetermined binding sites.

When determining the density of antibody bound to the inner surface of the polyolefinic culture bag, the number of antibody molecules /cm² could be determined for APTMS - modified, and DACH - modified polyolefinic culture bags, (left hand columns of Figure 9 B), in comparison to the non-specific binding of the antibody on non-modified polyolefinic surfaces (right hand columns). As a further negative control, TMOS - modified polyolefinic surfaces, which have no primary amino groups, were used. From the second negative control, it could be seen that in the absence of a reactive amino group, only a significantly reduced amount of antibody was bound.

For isolating cells, an alternative linkage group for binding of the cell surface binding ligand was used, i.e. the antibody was bound to an amino - modified polyolefinic vessel surface using protein G binding to the amino group introduced to the olefinic polymer.

As schematically shown in Figure 9 A), cells bearing a superficial antigen having binding specificity to the antibody that is bound to the polyolefinic surface will be linked to this surface, whereas cells without the specific superficial antigen don not bind and can be removed by washing, e.g. by an exchange of medium.

When using an antibody linked to a DACH - modified polyolefinic culture bag by recombinant protein G (available from Biovision), a specific cell type could be isolated from a mixture. The antibody was an anti-CD 20 antibody (Rituximab, Roche). As an example for a mixed cell population, an admixture of CD20-negative cells were loaded with red fluorescent dye (cell line supT1, dyed with Cell Tracker Orange, Invitrogen), and CD20-positive cells (cell line T2), labelled with green fluorescent marker (CFDA-SE, Invitrogen) were incubated in the culture bag for 45 minutes. Preferably, the incubation was at 0 to 5 °C. A micrograph is shown in Figure 9B, showing the statistic distribution of both cell types. Subsequently, the culture bag was washed using buffer or culture medium, resulting in an effective removal of the CD 20-negative cells, whereas the CD20-positive T2-cells remain bound to the antibody linked to the culture bag surface as shown in the micrograph of Figure 9C.

Negative controls without protein G or without antibody demonstrated that both cell types were effectively removed upon washing.

### Example 6: Modification and differentiation of adherent cells

As a modification of the above example, a cell surface binding ligand, e.g. a cell type specific antibody was bound to the culture vessel in admixture with viral particles, which were also bound to the culture vessel surface. As a result, both the selection of cells bearing the antigen for which the antibody has specificity was obtained, and the transfection of the selected cell type by the viral particles.

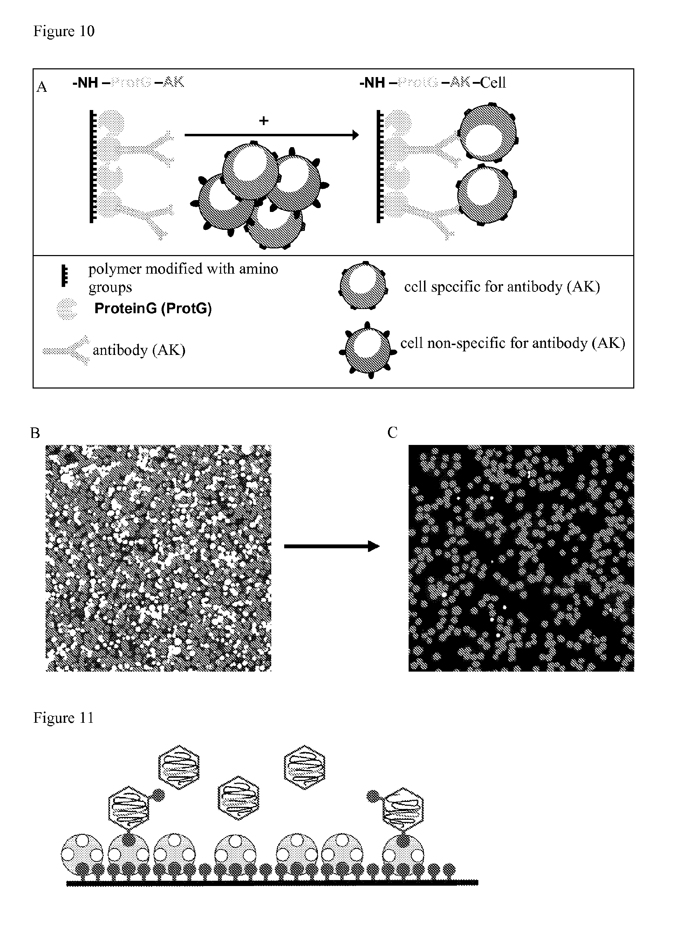

As an example for genetically modifying cells, optionally in combination with the selection of cells from an admixture of cells, in a process of the invention, transfection of cells adhering to the surface of the culture vessel is shown. As schematically shown in Figure 10, recombinant adenoviral particles were bound to the culture vessel surface, e.g. by streptavidin coupled to the biotin linked to amino groups introduced to the polyolefinic surface as described above. The free binding sites of the tetrameric streptavidin allowed binding of biotinylated viral particles. In this example, the viral particles were adenoviral particles containing an expression cassette for a fluorescent protein.

After binding viral particles to the surface of the culture vessel, unbound viral particles were removed by washing with buffer (PBS) or medium. In this experiment, an admixture of adenoviral particles containing an expression cassette for GFP (row A in Figure 11, upper particle), or Red (Figure 11, row A, lower particle) were used. Following removal of unbound viral particles, HEK293 cells were introduced into the culture bag in suspension. As shown in Figure 11, column 1, the non - biotinylated viral particles did not result in the transfection of cells, as in column 1, rows B and C, respectively, no fluorescent cell could be detected.

When the viral particle encoding Red was used in admixture with a non - biotinylated viral particle encoding GFP as shown in column 2, no green fluorescent cells (row B), but red fluorescent cells (row C) were generated.

When using biotinylated viral particle encoding GFP in admixture with non-biotinylated viral particles encoding Red, as shown in the column 3, cultivation of the cells resulted in green fluorescent cells (row B), but no red fluorescent cells (row C). This demonstrates that the linkage groups effectively and selectively bound the viral particle such that only the biotinylated adenoviral particle transfected the cultivated cells.

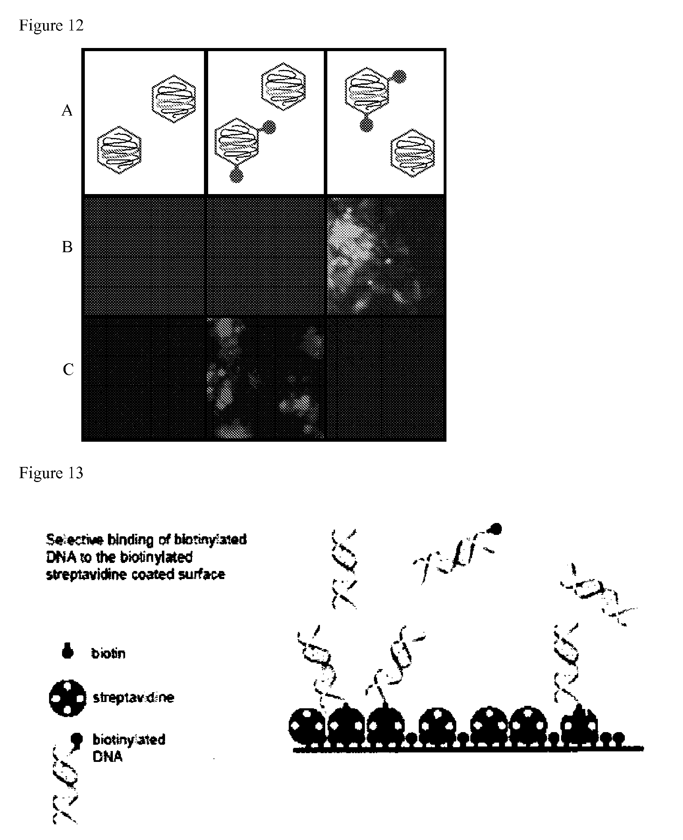

In the alternative to viral particles containing DNA which includes a transgene bound to the surface of the culture vessel, cells adhering to the culture vessel could be genetically manipulated, e.g. transformed, by uptake of DNA that was linked to the culture vessel. Figure 13 shows that in an embodiment of the process of the invention, DNA is linked to the surface of the culture vessel using a linkage group or compound. As an example for a linkage group, streptavidine that is bound to the surface-modified polyolefinic vessel by a biotin, is linked to biotinylated DNA containing a transgene. In detail, biotinylated DNA containing a mammalian expression cassette for GFP could be contacted with a streptavidin-coated polyolefinic culture bag. Following incubation, the buffer including non-biotinylated DNA could be removed.

Contacting cells in suspension with the culture vessel and incubation results in the adherence and growth of cells adherent to the vessel. Adherent cell growth occurs concurrent with uptake of the DNA into cells, e.g. resulting in transfection of the adherent cells.

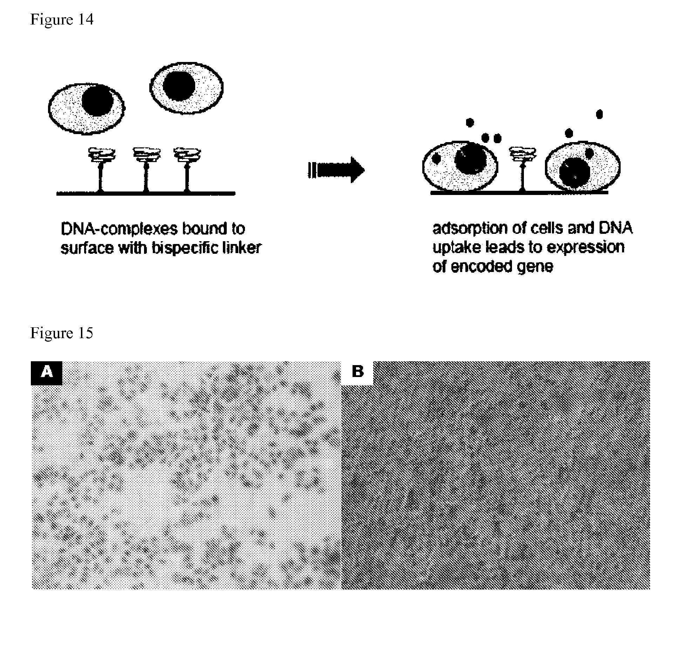

Schematically, Figure 14 depicts DNA bound to the surface of the cultivation vessel by a bispecific linker molecule, one end bound to the DNA, the other end bound to the modified vessel surface. From the cells added in suspension, the cells that adhere to the vessel surface, e.g. by specific adsorption to a cell-surface binding ligand (not shown), come into contact with the surface-bound DNA, resulting in uptake of the DNA. Genetic manipulation by the transgene is depicted as the translation and liberation of a transgene product.

Preferably, an antibody having specificity to a cell surface antigen was linked to the surface of the culture vessel, e.g. as described above using linkage groups, resulting in the preferential binding and isolation of the cell-type bearing the antigen, in combination with the genetic manipulation by uptake of the DNA linked to the vessel's surface.

### Example 7: Differentiation and cultivation of cells

For the differentiation of a cell type isolated from an admixture of cells, a cell surface binding ligand, e.g. a cell type specific antibody was linked to the surface of the culture vessel as described above, and a differentiation factor, e.g. GM-CSF in combination with IL4, BMP2, or at least one osteoinductive factor (e.g. β-glycerolphosphate, ascorbic acid and dexamethasone) was introduced to the culture medium during the cultivation of the isolated cells. In the alternative, a differentiation factor was linked to the culture vessel surface, e.g. using linkage groups, e.g. streptavidin in combination with a biotinylated differentiation factor.

It could be shown that the isolation of a cell type was achieved in combination with the differentiation of the isolated cell type as effected by the differentiation factor.

According to the invention, the isolated cell type was cryopreserved following differentiation and cultivation, resulting in a viable, cryopreserved cell layer of differentiated cells of one cell type.

Figure 15 shows micrographs of osteogenic cells which were cultivated from a cell suspension to an adherent cell layer. Figure 15A shows the osteogenic cells with dyeing by alizarin S for calcium. Cells were cultivated in osteogenic medium containing β-glycerol-2-phosphate, ascorbic acid and dexamethasone, which leads to cell differentiation and to the deposition of calcium within the extracellular matrix. Cultivation in medium without differentiation factor is shown in Figure 15B, wherein no differentiation is observed. This example shows that cultivation of adherently growing cells with concurrent differentiation is feasible, with cryopreservation of the differentiated adherent cell layer after addition of cryoprotectant. Thawing of cryopreserved differentiated adherent cell layers could be obtained as well as further cultivation after removal of cryoprotectant.

## Claims

1. Process for cultivating and cryopreserving mammalian adherent cells by the steps of providing a polyolefinic cell culture vessel with positively charged and/or hydrophilic substituent groups,
contacting cells suspended in cell culture medium with the cell culture vessel, cultivating the cells in the cell culture vessel under cell culture conditions, **characterized by**
adding a cryoprotectant to the cell culture medium, and
cooling the cultivated cells in the presence of the cryoprotectant to freezing temperatures.

2. Process according to claim 1, **characterized in that** after freezing, the cultivated cell layer is thawed,
the cryoprotectant is effectively removed, and the cell layer is incubated at cell culture conditions.

3. Process according to one of the preceding claims, **characterized in that** a cell surface binding ligand is bound to the surface of the culture vessel.

4. Process according to claim 3, **characterized in that** the cell surface binding ligand is selected from the group comprising antibodies, antibodies specific for a cell type, natural or synthetic ligands to a cell surface receptor, and cell surface receptors having affinity to a cell surface component.

5. Process according to one of the preceding claims, **characterized in that** following the contacting of cells with the cell culture vessel, the cells are incubated and the cell culture medium is removed and replaced by cell - free culture medium.

6. Process according to one of the preceding claims, **characterized in that** the cultivated cells are of ectodermal, endodermal or mesodermal origin.

7. Process for isolating, cultivating and preserving adherent mammalian cells in one cell culture vessel by the steps of
providing a polyolefinic cell culture vessel with positively charged and/or hydrophilic substituent groups,
binding a cell surface binding ligand to the surface of the culture vessel,
contacting cells suspended in cell culture medium with the cell culture vessel, removing cell culture medium including unbound cells,
adding cell - free culture medium,
cultivating the cells in the cell culture vessel under cell culture conditions to a state of adherent growth, **characterized by**
adding a cryoprotectant and cooling to freezing temperatures.

8. Process according to claim 7, **characterized in that** the cell surface binding ligand is selected from the group comprising antibodies, antibodies specific for a cell type, natural or synthetic ligands to a cell surface receptor, and cell surface receptors having affinity to a cell surface component.

9. Process according to one of the preceding claims, wherein during cultivation, a differentiation factor is added to the medium.

10. Process according to one of the preceding claims, **characterized in that** the cultivated cells are human stem cells excluding embryonic stem cells.

11. Process according to claim 10, **characterized in that** the human stem cells are selected from bone marrow stem cells, hematopoietic stem cells, endothelial stem cells and neuronal stem cells.

12. Process according to one of the preceding claims for the production of cryopreserved adherent cells.

13. Use of the process according to one of the preceding claims for the manufacture of an implant comprising the cultivated cell layer.

## Patentansprüche

1. Verfahren zum Kultivieren und Gefrierkonservieren von adhärenten Säugetierzellen durch die Schritte des Bereitstellens eines polyolefinischen Zellkulturgefäßes mit positiv geladenen und oder hydrophilen Substituentengruppen,
Kontaktieren der im Zellkulturmedium suspendierten Zellen mit dem Zellkulturgefäß, Kultivieren der Zellen in dem Zellkulturgefäß unter Zellkulturbedingungen, **gekennzeichnet durch**
Zugeben eines Gefrierschutzmittels zu dem Zellkulturmedium und Kühlen der kultivierten Zellen in der Gegenwart des Gefrierschutzmittels auf Gefriertemperaturen.

2. Verfahren nach Anspruch 1, **dadurch gekennzeichnet, dass** nach dem Gefrieren die kultivierte Zellschicht getaut wird, das Gefrierschutzmittel wirksam entfernt wird und die Zellschicht bei Zellkulturbedingungen inkubiert wird.

3. Verfahren nach einem der voranstehenden Ansprüche, **dadurch gekennzeichnet, dass** ein die Zelloberfläche bindender Ligand an die Oberfläche des Kulturgefäßes gebunden ist.

4. Verfahren nach Anspruch 3, **dadurch gekennzeichnet, dass** der die Zelloberfläche bindende Ligand aus der Gruppe ausgewählt ist, die Antikörper, für einen Zelltyp spezifische Antikörper, natürliche oder synthetische Liganden für einen Zelloberflächenrezeptor und Zelloberflächenrezeptoren mit Affinität für einen Zelloberflächenbestandteil umfasst.

5. Verfahren nach einem der voranstehenden Ansprüche, **dadurch gekennzeichnet, dass** im Anschluss an das Kontaktieren der Zellen mit dem Zellkulturgefäß die Zellen inkubiert werden und das Zellkulturmedium entfernt und durch ein zellfreies Kulturmedium ersetzt wird.

6. Verfahren nach einem der voranstehenden Ansprüche, **dadurch gekennzeichnet, dass** die kultivierten Zellen ektodermalen, endodermalen oder mesodermalen Ursprungs sind.

7. Verfahren zum Isolieren, Kultivieren und Konservieren adhärenter Säugetierzellen in einem Zellkulturgefäß durch die Schritte des
Bereitstellens eines polyolefinischen Zellkulturgefäßes mit positiv geladenen und/oder hydrophilen Substituentengruppen,
Bindens eines eine Zelloberfläche bindenden Liganden an die Oberfläche des Kulturgefäßes,
Kontaktierens der in dem Zellkulturmedium suspendierten Zellen mit dem Zellkulturgefäß,
Entfernens des Zellkulturmediums einschließlich ungebundener Zellen,
Zugebens zellfreien Kulturmediums,
Kultivierens der Zellen in dem Zellkulturgefäß unter Zellkulturbedingungen zu einem Zustand adhärenten Wachstums, **gekennzeichnet durch**
Zugeben eines Gefrierschutzmittels und Kühlen auf Gefriertemperaturen.

8. Verfahren nach Anspruch 7, **dadurch gekennzeichnet, dass** der die Zelloberfläche bindende Ligand aus der Gruppe ausgewählt ist, die Antikörper, für einen Zelltyp spezifische Antikörper, natürliche oder synthetischen Liganden für einen Zelloberflächenrezeptor und Zelloberflächenrezeptoren mit Affinität für einen Zelloberflächenbestandteil umfasst.

9. Verfahren nach einem der voranstehenden Ansprüche, wobei während der Kultivierung dem Medium ein Differenzierungsfaktor zugegeben wird.

10. Verfahren nach einem der voranstehenden Ansprüche, **dadurch gekennzeichnet, dass** die kultivierten Zellen menschliche Stammzellen ausgenommen embryonale Stammzellen sind.

11. Verfahren nach Anspruch 10, **dadurch gekennzeichnet, dass** die menschlichen Stammzellen unter Knochenmarkstammzellen, hematopoetischen Stammzellen, Endothelstammzellen und neuronalen Stammzellen ausgewählt sind.

12. Verfahren nach einem der voranstehenden Ansprüche zur Herstellung gefrierkonservierter adhärenter Zellen.

13. Verwendung des Verfahrens nach einem der voranstehenden Ansprüche für die Herstellung eines Implantats, das die kultivierte Zelleschicht umfasst.

## Revendications

1. Procédé de mise en culture et de cryoconservation de cellules adhérentes de mammifère comprenant les étapes suivantes :
fourniture de groupes de substituants chargés positivement et/ou hydrophiles dans un récipient de culture de cellules polyoléfiniques,
mise en contact des cellules en suspension dans le milieu de culture de cellules avec le récipient de culture de cellules,
mise en culture des cellules dans le récipient de culture de cellules, dans des conditions de culture de cellules,
**caractérisé par**
l'ajout d'un cryoprotecteur au milieu de culture de cellules, et
refroidissement des cellules cultivées en présence du cryoprotecteur jusqu'à des températures de congélation.

2. Procédé selon la revendication 1, **caractérisé en ce que**, après congélation, la couche de cellules cultivées est décongelée,
le cryoprotecteur est effectivement retiré, et la couche de cellules est mise à incuber dans des conditions de culture de cellules.

3. Procédé selon l'une des revendications précédentes, **caractérisé en ce qu'**un ligand de liaison de surface de cellule est lié à la surface du récipient de culture.

4. Procédé selon la revendication 3, **caractérisé en ce que** le ligand de liaison de surface de cellule est choisi dans le groupe comprenant les anticorps, les anticorps spécifiques à un type de cellule, les ligands naturels ou synthétiques d'un récepteur de surface de cellule, et les récepteurs de surface de cellule ayant une affinité avec un composant de surface de cellule.

5. Procédé selon l'une des revendications précédentes, **caractérisé en ce que**, à la suite de la mise en contact des cellules avec le récipient de culture de cellules, les cellules sont mises à incuber et le milieu de culture de cellules est retiré et remplacé par un milieu de culture exempt de cellules.

6. Procédé selon l'une des revendications précédentes, **caractérisé en ce que** les cellules cultivées sont d'origine ectodermique, endodermique ou mésodermique.

7. Procédé d'isolation, de mise en culture et de conservation de cellules adhérentes de mammifère dans un récipient de culture de cellules comprenant les étapes suivantes :
fourniture de groupes de substituants chargés positivement et/ou hydrophiles dans un récipient de culture de cellules polyoléfiniques,
liaison d'un ligand de liaison de surface de cellule à la surface du récipient de culture,
mise en contact des cellules en suspension dans le milieu de culture de cellules avec le récipient de culture de cellules,
retrait du milieu de culture de cellules comprenant des cellules non liées,
ajout d'un milieu de culture exempt de cellules,
mise en culture des cellules dans le récipient de culture de cellules dans des conditions de culture de cellules, pour obtenir un état de croissance adhérente, **caractérisé par**
l'ajout d'un cryoprotecteur et refroidissement jusqu'à des températures de congélation.

8. Procédé selon la revendication 7, **caractérisé en ce que** le ligand de liaison de surface de cellule est choisi dans le groupe comprenant les anticorps, les anticorps spécifiques à un type de cellule, les ligands naturels ou synthétiques d'un récepteur de surface de cellule, et les récepteurs de surface de cellule ayant une affinité avec un composant de surface de cellule.

9. Procédé selon l'une des revendications précédentes, dans lequel, au cours de la culture, un facteur de différenciation est ajouté au milieu.

10. Procédé selon l'une des revendications précédentes, **caractérisé en ce que** les cellules cultivées sont des cellules souches humaines, à l'exclusion des cellules souches embryonnaires.

11. Procédé selon la revendication 10, **caractérisé en ce que** les cellules souches humaines sont choisies parmi les cellules souches de moelle osseuse, les cellules souches hématopoïétiques multipotentes, les cellules souches endothéliales et les cellules souches neuronales.

12. Procédé, selon l'une des revendications précédentes, de production de cellules adhérentes cryoconservées.

13. Utilisation du procédé, selon l'une des revendications précédentes, pour la fabrication d'un implant comprenant la couche de cellules cultivées.
